(19) Europäisches Patentamt
European Patent Office
Office européen des brevets

(11) Veröffentlichungsnummer: **0 016 729**
**B1**

(12) # EUROPÄISCHE PATENTSCHRIFT

(45) Veröffentlichungstag der Patentschrift: **25.07.84**

(51) Int. Cl.³: **C 07 C 127/22**, C 07 C 87/60, A 01 N 47/34

(21) Anmeldenummer: **80810086.1**

(22) Anmeldetag: **07.03.80**

(54) **Substituierte N-(p-Aminophenyl)-N'-benzoylharnstoffe, Verfahren zu ihrer Herstellung, diese Verbindungen enthaltende Mittel und ihre Verwendung zur Bekämpfung von Schädlingen; alkenylsubstituierte p-Amino-anilinderivate.**

(30) Priorität: **13.03.79 CH 2379/79**
**12.02.80 CH 1134/80**

(43) Veröffentlichungstag der Anmeldung: **01.10.80 Patentblatt 80/20**

(45) Bekanntmachung des Hinweises auf die Patenterteilung: **25.07.84 Patentblatt 84/30**

(84) Benannte Vertragsstaaten: **BE CH DE FR GB IT NL**

(56) Entgegenhaltungen:
**JOURNAL OF AGRICULTURAL AND FOOD CHEMISTRY, Band 21, Nr. 3, Mai/Juni 1973, Seiten 348-354. American Chemical Society N.W. WASHINGTON D.C., USA WELLINGA et al. "Synthesis and Laboratory Evaluation of 1-(2,6-Disubstituted benzoyl)-3-phenylureas, a New Class of Insecticides. I. 1-(2,6-Dichlorobenzoyl)-3-phenylureas"**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Postfach**
**CH-4002 Basel (CH)**

(72) Erfinder: **Ehrenfreund, Josef Dr.**
**Langenhagweg 11**
**CH-4123 Allschwil (CH)**

**EP 0 016 729 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte N-(p-Aminophenyl)-N'-benzoylharnstoffe, Verfahren zu ihrer Herstellung und ihre Verwendung in der Schädlingsbekämpfung.

Die erfindungsgemässen substituierten N-(p-Aminophenyl)-N'-benzoylharnstoffe haben die Formel I

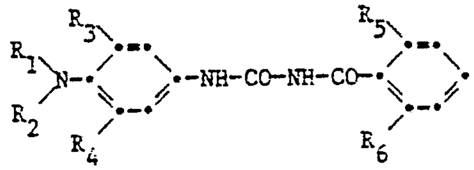

(I)

worin

$R_1$ und $R_2$ unabhängig voneinander $C_1$-$C_4$-Alkyl oder $C_3$-$C_5$-Alkenyl ;

$R_3$ und $R_4$ unabhängig voneinander Chlor oder Brom ; und $R_5$ und $R_6$ unabhängig voneinander Wasserstoff, Fluor oder Chlor bedeuten, mit der Massgabe, dass die Reste $R_5$ und $R_6$ nicht beide gleichzeitig Wasserstoff bedeuten.

Wegen ihrer Wirkung als Schädlingsbekämpfungsmittel bevorzugt sind Verbindungen der Formel I, die dadurch gekennzeichnet sind, dass $R_3$ und $R_4$ Chlor bedeuten. Hervorzuheben sind daneben solche Verbindungen der Formel I, worin $R_5$ und $R_6$ Fluor bedeuten. Wertvoll sind aufgrund ihrer biologischen Wirksamkeit ferner die Verbindungen der Formel I, worin mindestens einer der Reste $R_1$ und $R_2$ Allyl bedeutet. Besonders wirksam sind die erfindungsgemässen Verbindungen der Formel I, bei denen einer der Reste $R_1$ und $R_2$ Allyl und der andere $C_1$-$C_4$-Alkyl bedeutet.

Die Verbindungen der Formel I können nach an sich bekannten Verfahren hergestellt werden (vgl. u. a. die deutschen Offenlegungsschriften Nr. 2.123.236, 2.601.780).

So kann man z. B. eine Verbindung der Formel I erhalten durch Umsetzung

a) einer Verbindung der Formel II

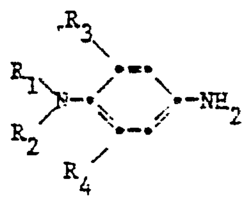

(II)

mit einer Verbindung der Formel III

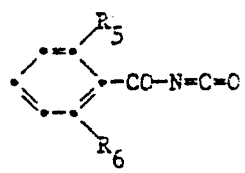

(III)

oder

b) einer Verbindung der Formel IV

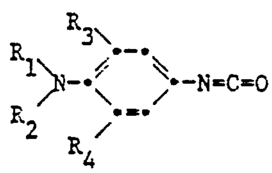

(IV)

mit einer Verbindung der Formel V

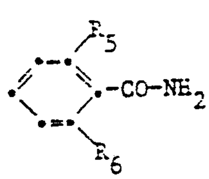

(V)

In den obigen Formeln II, III, IV und V haben die Reste $R_1$, $R_2$, $R_3$, $R_4$, $R_5$ und $R_6$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z. B. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z. B. Aceton, Methyläthylketon, Methylisopropylketon und Methylisobutylketon. Verfahren a) wird im allgemeinen bei einer Temperatur von − 10 bis 100 °C, vorzugsweise zwischen 15 und 25 °C, gegebenenfalls in Gegenwart einer organischen Base, z. B. Triäthylamin, durchgeführt. Die Durchführung von Verfahren b) erfolgt bei einer Temperatur von 0 bis 120 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der vorstehenden Formeln II, III, IV und V sind bekannt oder lassen sich, falls sie neu sind, analog bekannten Verfahren herstellen. So können die p-Phenylendiamine der Formel II durch N-Alkylierung bzw. N-Alkenylierung der entsprechenden p-Nitroaniline und nachfolgende Reduktion oder katalytische Hydrierung der Nitrogruppe zur Aminogruppe hergestellt werden [vgl. z. B. Rec. *21,* 271 (1902) ; J. Am. Soc. *68,* 1604 (1946) ; J. Org. Chem. *11,* 378 (1946) ; Rec. *79,* 995 (1970)]. Die Isocyanate der Formel IV sind durch Phosgenisierung der entsprechenden N,N-substituierten p-Phenylendiamine der Formel II nach allgemein üblichen Arbeitsweisen erhältlich. Zu den Verbindungen der Formel III kann man wie folgt gelangen (vgl. J. Agr. Food Chem. *21(3),* 348-993 ; 1973) :

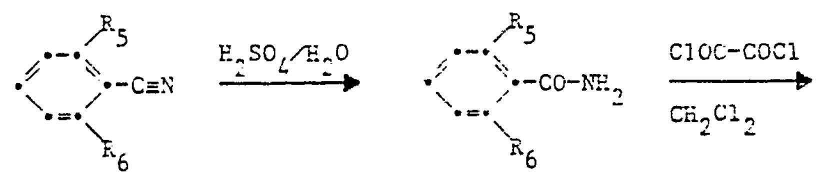


(III)

In obigen Formeln haben $R_5$ und $R_6$ die unter Formel I angegebenen Bedeutungen.

Diejenigen Verbindungen der Formel II, worin mindestens einer der Reste $R_1$ oder $R_2$ einen $C_3$-$C_5$-Alkenylrest darstellt, sind neu und können mit Hilfe der oben erwähnten Verfahren hergestellt werden. Die neuen Ausgangsverbindungen, welche zu den wertvollen Schädlingsbekämpfungsmittel der Formel I führen, bilden ebenfalls einen Gegenstand der vorliegenden Erfindung.

Es ist bereits bekannt, dass bestimmte N-Phenyl-N'-benzoylharnstoffe insektizide Eigenschaften besitzen (vgl. deutsche Offenlegungsschriften 2.123.236, 2.504.982, 2.537.413, 2.601.780 und 2.726.684, die belgischen Patentschriften 832.304, 843.906, 844.066 und 867.046 sowie die US-Patentschrift 4.089.975). Aus J. Agr. Food Chem. *21,* No. 3, 348 ff. (1973) sind weiterhin substituierte N-Phenyl-N'-2,6-dichlorbenzoylharnstoffe bekannt, die insektizide Eigenschaften aufweisen sollen. Auf Seite 353 dieser Veröffentlichung werden entsprechende N-(4-Dimethylamino)-phenyl- und N-(3-Chlor-4-dimethylamino)-phenylderivate erwähnt, die jedoch — wie aus der dort aufgeführter Tabelle III ersichtlich — nur unzureichende Insektizidwirkung zeigen.

Ueberraschenderweise wurde demgegenüber gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen : Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer sehr günstigen Wirkung gegenüber Fliegen, wie z. B. Musca domestica, und Mückenlarven eignen sich Verbindungen der Formel I auch zur Bekämpfung von pflanzenschädigenden Frassinsekten, in Zier- und Nutzpflanzen, insbesondere in Baumwollkulturen (z. B. gegen Spodoptera littoralis und Heliothis virescens) sowie in Gemüsekulturen (z. B. gegen Leptinotarsa decemlineata und Pieris brassicae). Hervorzuheben ist besonders die ovizide bzw. ovolarvizide Wirkung von Verbindungen der Formel I. Werden Verbindungen der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z. B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z. B. folgende Wirkstoffe in Betracht :

organische Phosphorverbindungen,
Nitrophenole und Derivate,
Formamidine, Harnstoffe,
Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u. a. : Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithiote.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z. B. natürlich oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind « cattle dips », d. h. Viehbäder, und « spray races », d. h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden :

Feste Aufarbeitungsformen :
Stäubemittel, Streumittel,
Granulate (Umhüllungsgranulate, Imprägnierungsgranulate und Homogengranulate) ;

Flüssige Aufarbeitungsformen :
a) in Wasser dispergierbare Wirkstoffkonzentrate : Spritzpulver (wettable powders), Pasten, Emulsionen ;
b) Lösungen.

Der Gehalt an Wirkstoff in den oben beschriebenen Mitteln liegt zwischen 0,1 bis 95 Gew.-%.

Die Wirkstoffe der Formel I können beispielsweise wie folgt formuliert werden :

Stäubemittel : Zur Herstellung eines a) 5 %igen und b) 2 %igen Stäubemittels werden die folgenden Stoffe verwendet :

a) 5 Teile Wirkstoff,
95 Teile Talkum ;
b) 2 Teile Wirkstoff,
1 Teil hochdisperse Kieselsäure,
97 Teile Talkum.

Die Wirkstoffe werden mit den Trägerstoffen vermischt und vermahlen.

Granulat : Zur Herstellung eines 5 %igen Granulates werden die folgenden Stoffe verwendet :

5 Teile Wirkstoff,
0,25 Teile epoxydiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz wird mit dem epoxydierten Pflanzenöl in 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver : Zur Herstellung eines a) 40 %igen, b) und c) 25 %igen, d) 10 %igen Spritzpulvers werden folgende Bestandteile verwendet :
a) 40 Teile Wirkstoff,
5 Teile Ligninsulfonsäure-Natriumsalz,

1 Teil Dibutylnaphthalinsulfonsäure-Natriumsalz,
54 Teile Kieselsäure ;

b) 25 Teile Wirkstoff,
4,5 Teile Calcium-Ligninsulfonat,
1,9 Teile Champagne-Kreide/Hydroxyäthylcellulose-Gemisch (1 : 1),
1,5 Teile Natrium-dibutyl-naphthalinsulfonat,
19,5 Teile Kieselsäure,
19,5 Teile Champagne-Kreide,
28,1 Teile Kaolin ;

c) 25 Teile Wirkstoff,
2,5 Teile Isooctylphenoxy-polyoxyäthylen-äthanol,
1,7 Teile Champagne-Kreide/Hydroxyäthylcellulose-Gemisch (1 : 1),
8,3 Teile Natriumaluminiumsilikat,
16,5 Teile Kieselgur,
46 Teile Kaolin ;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate : Zur Herstellung eines a) 10 %igen, b) 25 %igen und c) 50 %igen emulgierbaren Konzentrates werden folgende Stoffe verwendet :

a) 10 Teile Wirkstoff
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40 Teile Dimethylformamid,
43,2 Teile Xylol ;

b) 25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol ;

c) 50 Teile Wirkstoff,
4,2 Teile Tributylphenol-Polyglykoläther,
5,8 Teile Calcium-Dodecylbenzolsulfonat,
20 Teile Cyclohexanon
20 Teile Xylol.

Aus solchen Konzentrationen können durch Verdünnen mit Wasser Emulsionen jeder gewünschten Konzentration hergestellt werden.

Sprühmittel : Zur Herstellung eines a) 5 %igen und b) 95 %igen Sprühmittels werden die folgenden Bestandteile verwendet :

a) 5 Teile Wirkstoff,
1 Teil epoxydiertes Pflanzenöl,
94 Teile Benzin (Siedegrenzen 160-190 °C) ;

b) 95 Teile Wirkstoff,
5 Teile epoxydiertes Pflanzenöl.

Beispiel 1

4,5 g (0.0175 Mol) 3,5-Dichlor-4-N,N-diallylamino-anilin werden in absolutem Aether gelöst und unter Kühlung und Ausschluss von Feuchtigkeit mit 3,5 g 2,6-Difluorbenzoylisocyanat versetzt. Der nach

einiger Zeit ausgefallene Niederschlag wird abgesaugt und aus Aethanol umkristallisiert. Man erhält N[1]-[3,5-Dichlor-4-N,N-diallylamino]-phenyl-N[2]-2,6-difluorbenzoylharnstoff vom Schmelzpunkt 150-151,5 °C.

Analog den vorstehend beschriebenen Arbeitsweisen werden die folgenden Verbindungen der Formel I hergestellt :

| $R_1$ | $R_2$ | $R_3$ | $R_4$ | $R_5$ | $R_6$ | Schmelzpunkt [°C] |
|---|---|---|---|---|---|---|
| $CH_3$ | $CH_3$ | Cl | Cl | F | F | 209-211 |
| $CH_3$ | $CH_3$ | Cl | Cl | H | Cl | 189-190 |
| $CH_3$ | $CH_3$ | Cl | Cl | Cl | F | 194-196 |
| $CH_3$ | $CH_3$ | Cl | Cl | Cl | Cl | 209-211 |
| $CH_3$ | $CH_3$ | Cl | Cl | H | F | 203-204 |
| $CH_3$ | $CH_3$ | Br | Br | F | F | 215-219 |
| $CH_3$ | $CH_3$ | Br | Br | H | Cl | 208-210 |
| $CH_3$ | $CH_3$ | Br | Br | Cl | Cl | |
| $CH_3$ | $-CH_2-CH=CH_2$ | Cl | Cl | F | F | 146-148 |
| $CH_3$ | $-CH_2-CH=CH_2$ | Cl | Cl | Cl | Cl | 172-175 |
| $CH_3$ | $-CH_2-CH=CH_2$ | Cl | Cl | H | F | 128-131 |
| $CH_3$ | $-CH_2-CH-CH_2$ | Cl | Cl | F | Cl | 163-166 |
| $CH_3$ | $-CH_2-CH=CH_2$ | Cl | Cl | H | Cl | 114-117 |
| $CH_3$ | $-CH_2-CH=CH_2$ | Cl | Br | F | F | 156.5-159 |
| $CH_3$ | $-CH_2-CH=CH_2$ | Cl | Br | H | F | |
| $CH_3$ | $-CH_2-CH=CH_2$ | Br | Br | F | F | 170-172 |
| $CH_3$ | $-CH_2-CH=CH_2$ | Br | Br | Cl | Cl | |
| $-CH_2-CH=CH_2$ | $-CH_2-CH=CH_2$ | Cl | Cl | H | Cl | 148-150 |
| $-CH_2-CH=CH_2$ | $-CH_2-CH=CH_2$ | Cl | Cl | F | Cl | 171-173 |
| $-CH_2-CH=CH_2$ | $-CH_2-CH=CH_2$ | Cl | Cl | Cl | Cl | 166-168 |
| $-CH_2-CH=CH_2$ | $-CH_2-CH=CH_2$ | Cl | Cl | H | F | 104-106 |
| $-CH_2-CH=CH_2$ | $-CH_2-CH=CH_2$ | Br | Br | F | F | 148-152 |
| $-CH_2-CH=CH_2$ | $-CH_2-CH=CH_2$ | Br | Br | H | Cl | 128-130 |
| $-CH_2-CH=CH_2$ | $-CH_2-CH=CH_2$ | Br | Br | Cl | Cl | 174-176 |
| $-CH_2-CH=CH_2$ | $-CH_2-CH=CH_2$ | Br | Br | H | F | 117-119 |
| $-CH_2-CH=CH_2$ | $-n-C_3H_7$ | Cl | Cl | F | F | 132-134 |
| $-CH_2-CH=CH_2$ | $-i-C_3H_7$ | Cl | Cl | H | Cl | |
| $-CH_2-CH=CH_2$ | $-n-C_4H_9$ | Cl | Cl | F | F | 127-130 |
| $-C_2H_5$ | $-CH_3$ | Cl | Cl | H | F | |
| $-n-C_3H_7$ | $-n-C_3H_7$ | Cl | Cl | F | F | 136-137 |
| $-n-C_4H_9$ | $-CH_3$ | Cl | Br | H | Cl | |
| $-n-C_4H_9$ | $-n-C_4H_9$ | Cl | Cl | F | F | 159-161 |
| $-(CH_2)_3-CH=CH_2$ | $-CH_3$ | Cl | Cl | F | F | |
| $-n-C_4H_9$ | $-CH_3$ | Cl | Cl | F | F | 149-152 |

Beispiel 2

Wirkung gegen Musca domestica :

Je 50 frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmte Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

Beispiel 3

Wirkung gegen Lucilia sericata :

Zu 9 ml eines Zuchtmediums wurde bei 50 °C 1 ml einer 0,5 % Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeigten in diesem Test gute Wirkung gegen Lucilia sericata.

Beispiel 4

Wirkung gegen Aëdes aegypti :

Auf die Oberfläche von 150 ml Wasser, das sich in einem Behälter befindet, wurde so viel einer 0,1 %igen acetonischen Lösung des Wirkstoffes pipettiert, dass Konzentrationen von je 10, 5 und 1 ppm erhalten wurden. Nach Verdunsten des Acetons wurde der Behälter mit 30-40 3-tägigen Aëdes-Larven beschickt. Nach 1, 2 und 5 Tagen wurde die Mortalität geprüft.

Verbindungen gemäss Beispiel 1 zeigten in diesem Test gute Wirkung gegen Aëdes aegypti.

Beispiel 5

Insektizide Frassgift-Wirkung :

Baumwollpflanzen wurden mit einer 0,05 %igen wässrigen Wirkstoffemulsion (erhalten aus einem 10 %igen emulgierbaren Konzentrat) besprüht.

Nach dem Antrocknen des Belages wurden die Baumwollpflanzen je mit Spodoptera littoralis- und Heliothis virescens-Larven im dritten larvalen Stadium besetzt. Der Versuch wurde bei 24 °C und 60 % relativer Luftfeuchtigkeit durchgeführt.

Verbindungen gemäss Beispiel 1 zeigten im obigen Test gute insektizide Frassgift-Wirkung gegen Spodoptera- und Heliothis-Larven.

Beispiel 6

Wirkung auf Spodoptera littoralis und Heliothis virescens (Larven und Eier) :

Es wurden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages wurden die eingetopften Pflanzen in ein Blechgefäss von etwa 20 Litern Inhalt gestellt, das mit einer Glasplatte agbedeckt wurde. Die Feuchtigkeit im Inneren des abgedeckten Gefässes wurde so reguliert, dass sich kein Kondenswasser bildete. Direktes, auf die Pflanzen fallendes Licht wurde vermieden. Dann wurden die drei Pflanzen infestiert, und zwar insgesamt mit :

a) 50 Larven von Spodoptera littoralis oder Heliothis virescens des ersten larvalen Stadiums ;

b) 20 Larven von Spodoptera littoralis oder Heliothis virescens des dritten larvalen Stadiums ;

c) zwei Eispiegeln von Spodoptera littoralis oder Heliothis virescens. Dazu wurden je 2 Blätter einer Pflanze in einem beidseitig mit Gaze verschlossenen Plexiglaszylinder eingeschlossen ; zwei Eispiegel

von Spodoptera oder ein Teil eines Baumwollblattes mit darauf abgelegten Eiern von Heliothis wurden zu den eingeschlossenen Blättern gegeben.

Nach 4 bis 5 Tagen erfolgte die Auswertung gegenüber unbehandelten Kontrollen unter Berücksichtigung folgender Kriterien :

a) Anzahl der noch lebenden Larven,
b) Larvale Entwicklungs- und Häutungshemmung,
c) Frasschaden (Schabfrass und Lochfrass),
d) Schlupfrate (Anzahl der aus den Eiern geschlüpften Larven).

Die Verbindungen gemäss Beispiel 1 zeigten gute Gesamt-Wirksamkeit in obigem Test.

Beispiel 7

Ovizide Wirkung auf Spodoptera littoralis :

Auf Filterpapier abgelegte Eier von Spodoptera littoralis wurden aus dem Papier ausgeschnitten und in eine 0,05 % Gew.-%ige Lösung des Wirkstoffes in einem Aceton-Wasser-Gemisch (1 : 1) getaucht. Die so behandelten Eiablagen wurden dann aus diesem Gemisch herausgenommen und bei 21 °C und 60 % relativer Feuchtigkeit in Kunststoffschalen deponiert.

Nach 3 bis 4 Tagen wurde die Schlupfrate, d. h. die Anzahl Larven, die sich aus den behandelten Eiern entwickelt hatten, bestimmt.

Verbindungen gemäss Beispiel 1 zeigten gute Wirkung im obigen Test.

Beispiel 8

Ovizide Wirkung auf Epilachna varivestis :

Es wurden 20 Gew.-% Wirkstoff, 70 Gew.-% Xylol und 10 Gew.-% einer Mischung aus einem Reaktionsprodukt eines Alkylphenoles mit Aethylenoxyd und Calcium-dodecylbenzolsulfonat miteinander vermischt. Aus diesem Konzentrat wurden wässrige Emulsionen enthaltend 800 und 1 600 ppm Wirkstoff hergestellt.

Jeweils ca. 100 auf Blätter von Phaseolus vulgaris frisch abgelegte Eier von Epilachna varivestis (mexikanischer Bohnenkäfer) wurden mit den oben beschriebenen wässrigen Emulsionen (Konzentration 800 bzw. 1 600 ppm Wirkstoff) angefeuchtet und leicht getrocknet.

In einem gelüfteten Gefäss wurden die behandelten Gelege solange gehalten, bis die gleichzeitig angesetzten unbehandelten Kontrollen geschlüpft waren. Unter einem Binocular erfolgte Auswertung hinsichtlich der erzielten prozentualen Abtötung.

Verbindungen gemäss Beispiel 1 zeigten gute Wirkung in obigem Test.

Beispiel 9

Ovizide Wirkung auf Heliothis virescens und Leptinotarsa decemlineata :

Entsprechende Mengenanteile einer benetzbaren pulverförmigen Formulierung, enthaltend 25 Gew.-% des zu prüfenden Wirkstoffes, wurden mit jeweils soviel Wasser vermischt, dass sich wässrige Emulsionen von ansteigender Wirkstoffkonzentration ergaben.

In diese wirkstoffhaltigen Emulsionen wurden eintägige Eigelege von Heliothis auf Cellophan bzw. Eigelege von Leptinotarsa auf Kartoffelblättern während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100 %-igen Abtötung der Eier erforderliche minimale Wirkstoffkonzentration bestimmt.

Verbindungen gemäss Beispiel 1 zeigten in diesem Test gute ovizide Wirkung gegen die geprüften Schädlinge.

Beispiel 10

Wirkung auf Laspeyresia pomonella (Eier) :

Abgelegte Eier von Laspeyresia pomonella, die nicht älter als 24 Stunden waren, wurden auf Filterpapier für 1 Minute in eine acetonischwässrige Lösung enthaltend 400 ppm des zu prüfenden Wirkstoffes eingetaucht. Nach dem Antrocknen der Lösung wurden die Eier in Petrischalen ausgelegt und bei einer Temperatur von 28 °C belassen. Nach 6 Tagen wurde der prozentuale Schlupf aus den behandelten Eiern bewertet. Verbindungen gemäss Beispiel 1 zeigte gute Wirkung in obigem Test.

Beispiel 11

Chemosterilisierende Wirkung auf Anthonomus grandis

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z. B. « Actamer B 100 ») desinfiziert und dann in Schalen, die eine geeignete Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 zeigten gute Wirkung im obigem Test.

**Ansprüche**

1. Verbindung der Formel

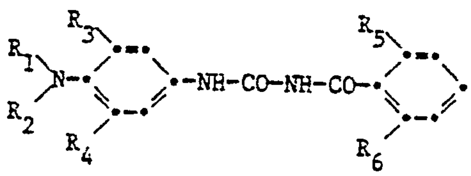

(I)

worin

$R_1$ und $R_2$ unabhängig voneinander $C_1$-$C_4$-Alkyl oder $C_3$-$C_5$-Alkenyl ;

$R_3$ und $R_4$ unabhängig voneinander Chlor oder Brom ; und $R_5$ und $R_6$ unabhängig voneinander Wasserstoff, Fluor oder Chlor bedeuten, mit der Massgabe, dass die Reste $R_5$ und $R_6$ nicht beide gleichzeitig Wasserstofff bedeuten.

2. Verbindung der Formel I gemäss Anspruch 1, dadurch gekennzeichnet, dass $R_3$ und $R_4$ Chlor bedeuten.

3. Verbindung der Formel I gemäss Anspruch 1 oder 2, dadurch gekennzeichnet, dass $R_5$ und $R_6$ Fluor bedeuten.

4. Verbindung der Formel I gemäss Anspruch 1 bis 3, dadurch gekennzeichnet, dass mindestens einer der Reste $R_1$ und $R_2$ Allyl bedeutet.

5. Verbindung der Formel I gemäss Anspruch 4, dadurch gekennzeichnet, dass einer der Reste $R_1$ und $R_2$ Allyl und der andere $C_1$-$C_4$-Alkyl bedeutet.

6. Verbindung gemäss Anspruch 5 der Formel

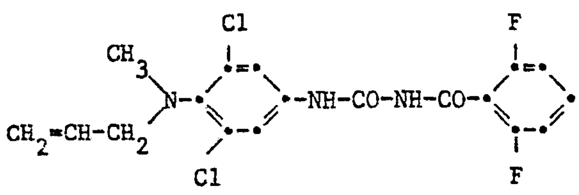

7. Verbindung gemäss Anspruch 5 der Formel

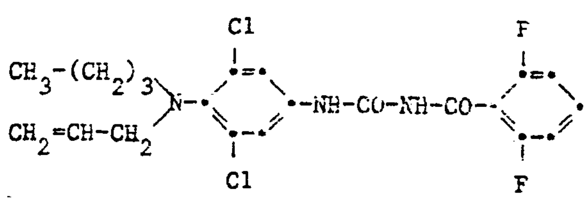

8. Verbindung gemäss Anspruch 3 der Formel

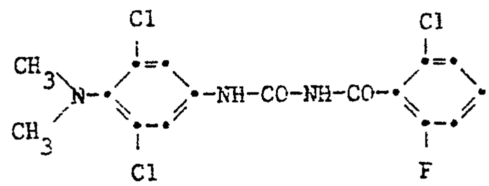

9. Verfahren zur Herstellung einer Verbindung gemäss den Ansprüchen 1 bis 8, dadurch gekennzeichnet, dass man

a) eine Verbindung der Formel II

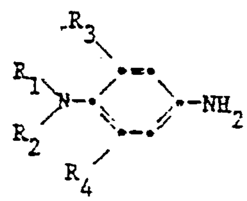

(II)

mit einer Verbindung der Formel III

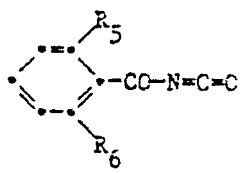

(III)

oder

b) eine Verbindung der Formel IV

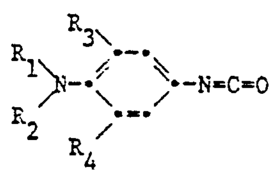

(IV)

mit einer Verbindung der Formel V

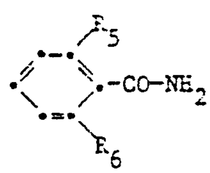

(V)

umsetzt, wobei in den Formeln II bis V die Reste $R_1$ bis $R_6$ die in den Ansprüchen 1 bis 5 angegebenen Bedeutungen haben.

10. Schädlingsbekämpfungsmittel, welches als aktive Komponente eine Verbindung gemäss den Ansprüchen 1 bis 8 zusammen mit geeigneten Trägern und/oder anderen Zuschlagstoffen enthält.

11. Verwendung von Verbindungen gemäss den Ansprüchen 1 bis 8 zur Bekämpfung von Schädlingen, vorzugsweise von Insekten und Vertretern der Ordnung Akarina.

12. Verwendung gemäss Anspruch 11 als Ovizide zur Bekämpfung pflanzenschädigender Insekten.

13. Verbindung der Formel IIa

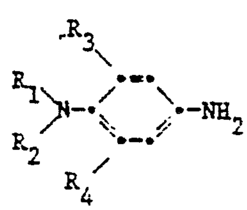

(IIa)

worin $R_1$, $R_2$, $R_3$ und $R_4$ die in Anspruch 1 angegebenen Bedeutungen haben, und wobei mindestens einer der Reste $R_1$ und $R_2$ $C_3$-$C_5$-Alkenyl bedeutet.

**Claims**

1. A compound of the formula

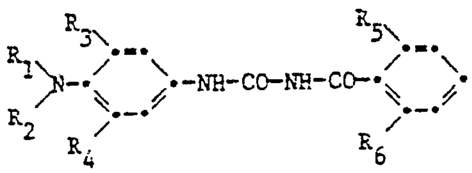

(I)

wherein

each of $R_1$ and $R_2$ independently is $C_1$-$C_4$ alkyl or $C_3$-$C_5$ alkenyl,

each of $R_3$ and $R_4$ independently is chlorine or bromine, and each of $R_5$ and $R_6$ independently is hydrogen, fluorine or chlorine, with the proviso that $R_5$ and $R_6$ are not both simultaneously hydrogen.

2. A compound of the formula I according to claim 1, wherein $R_3$ and $R_4$ are chlorine.

3. A compound of the formula I according to claim 1 or 2, wherein $R_5$ and $R_6$ are fluorine.

4. A compound of the formula I according to any one of claims 1 to 3, wherein at least one of $R_1$ and $R_2$ is allyl.

5. A compound of the formula I according to claim 4, wherein one of $R_1$ and $R_2$ is allyl and the other is $C_1$-$C_4$ alkyl.

6. A compound according to claim 5 of the formula

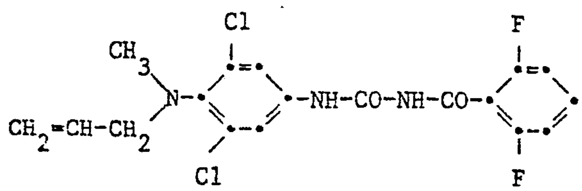

7. A compound according to claim 5 of the formula

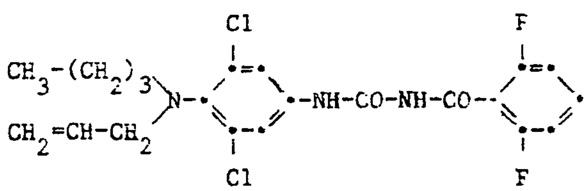

8. A compound according to claim 3 of the formula

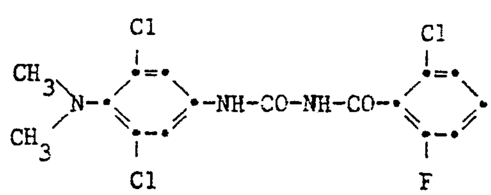

9. A process for the manufacture of a compound according to claims 1 to 8, which process comprises reacting

a) a compound of the formula II

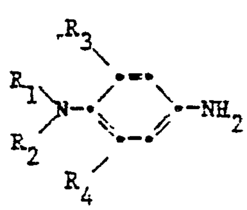

(II)

with a compound of the formula III

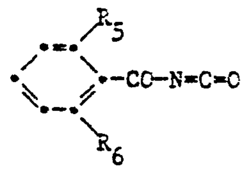

(III)

or

b) a compound of the formula IV

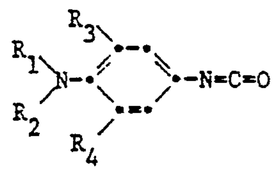

(IV)

with a compound of the formula V

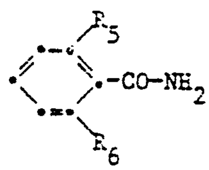

(V)

in which formulae II to V the symbols $R_1$ to $R_6$ are as defined in claims 1 to 5.

10. A pesticidal composition which contains, as active component, a compound according to any one of claims 1 to 8 together with suitable carriers and/or other adjuvants.

11. A method of controlling pests, preferably insects and representatives of the order Acarina, which comprises the use of a compound according to claims 1 to 8.

12. A method according to claim 11 for controlling plant-destructive insects, which comprises the use of a compound according to claims 1 to 8 as ovicide.

13. The compound of the formula IIa

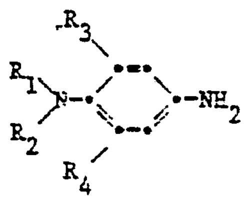

(IIa)

wherein $R_1$, $R_2$, $R_3$ and $R_4$ are as defined in claim 1, with the proviso that at least one of $R_1$ and $R_2$ is $C_3$-$C_5$ alkenyl.

**Revendications**

1. Composé de formule

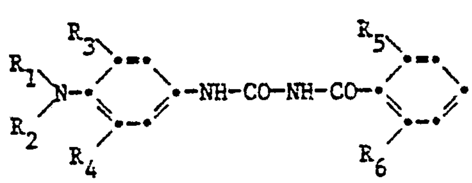

(I)

dans laquelle

$R_1$ et $R_2$ représentent chacun, indépendamment l'un de l'autre, un groupe alkyle en $C_1$-$C_4$ ou alcényle en $C_3$-$C_5$ ;

$R_3$ et $R_4$ représentent chacun, indépendamment l'un de l'autre, le chlore ou le brome ; et $R_5$ et $R_6$

représentent chacun, indépendamment l'un de l'autre, l'hydrogène, le fluor ou le chlore, étant spécifié que les symboles $R_5$ et $R_6$ ne peuvent représenter tous deux simultanément l'hydrogène.

2. Composé de formule I selon la revendication 1, caractérisé en ce que $R_3$ et $R_4$ représentent le chlore.

3. Composé de formule I selon la revendication 1 ou 2, caractérisé en ce que $R_5$ et $R_6$ représentent le fluor.

4. Composé de formule I selon les revendications 1 à 3, caractérisé en ce que l'un au moins des symboles $R_1$ et $R_2$ représente un groupe allyle.

5. Composé de formule I selon la revendication 4, caractérisé en ce que l'un des symboles $R_1$ et $R_2$ représente un groupe allyle et l'autre un groupe alkyle en $C_1$-$C_4$.

6. Composé selon la revendication 5 de formule

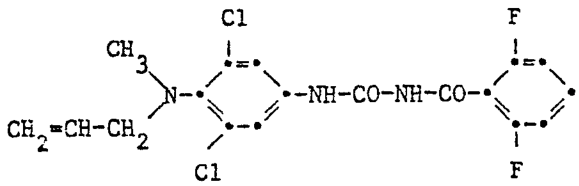

7. Composé selon la revendication 5 de formule

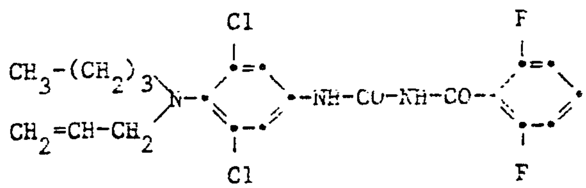

8. Composé selon la revendication 3 de formule

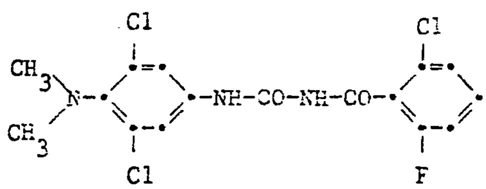

9. Procédé de préparation d'un composé selon les revendications 1 à 8, caractérisé en ce que

a) on fait réagir un composé de formule II

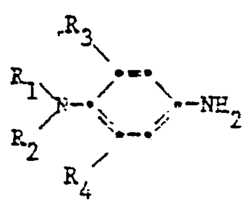

(II)

avec un composé de formule III

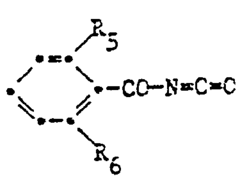

(III)

ou bien

b) on fait réagir un composé de formule IV

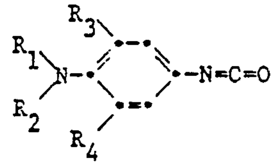

(IV)

avec un composé de formule V

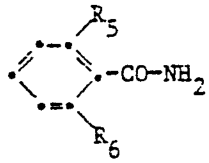

(V)

les symboles $R_1$ à $R_6$ ayant dans les formules II à V les significations indiquées dans les revendications 1 à 5.

10. Produit pesticide contenant en tant que composant actif un composé selon les revendications 1 à 8 avec des véhicules et/ou d'autres additifs appropriés.

11. Utilisation des composés selon les revendications 1 à 8 pour la lutte contre les parasites, de préférence les insectes et les représentants de l'ordre des acariens.

12. Utilisation selon la revendication 11 en tant qu'ovicides pour la lutte contre les insectes nuisibles pour les végétaux.

13. Composé de formule IIa

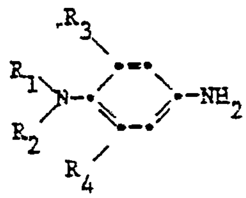

(IIa)

dans laquelle $R_1$, $R_2$, $R_3$ et $R_4$ ont les significations indiquées dans la revendication 1 et l'un au moins des symboles $R_1$ et $R_2$ représente un groupe alcényle en $C_3$-$C_5$.